# EUROPEAN PATENT APPLICATION

(11) **EP 3 583 945 A1**
(43) Date of publication of application: **25.12.2019**
(21) Application number: 18753797.2
(22) Date of filing: 09.02.2018
(51) Int. Cl.: A61K 35/28, A61K 45/00, A61P 11/00, A61P 43/00

(54) **PULMONARY FIBROSIS TREATMENT AGENT, PTPRR EXPRESSION-PROMOTING AGENT, AND KIT FOR PULMONARY FIBROSIS TREATMENT**

(30) Priority: 15.02.2017 JP 2017025790
(71) Applicant: Rohto Pharmaceutical Co., Ltd., Osaka-shi Osaka 544-8666 (JP); Public University Corporation Nara Medical University, Nara 634-8521 (JP)
(72) Inventor: FURUNO Tetsuo, Osaka-shi Osaka 544-8666 (JP); ITO Toshihiro, Kashihara-shi Nara 634-8521 (JP); KIMURA Hiroshi, Kashihara-shi Nara 634-8521 (JP); YOSHIKAWA Masanori, Kashihara-shi Nara 634-8521 (JP); SAWADA Makiko, Kashihara-shi Nara 634-8521 (JP)
(74) Representative: Mewburn Ellis LLP
(86) International application number: PCT/JP2018/004623
(87) International publication number: WO 2018/151046

(57) **Abstract**

The purpose of the present invention is to provide a novel treatment agent for pulmonary fibrosis for which there is currently no effective drug therapy established. The present invention is a therapeutic agent for pulmonary fibrosis containing mesenchymal stem cells. The pulmonary fibrosis is preferably usual interstitial pneumonia (UIP) or idiopathic pulmonary fibrosis (IPF). Also, the mesenchymal stem cells are preferably derived from adipose tissue or are allogeneic.

## Description

### TECHNICAL FIELD

The present invention relates to a Therapeutic agent for Pulmonary fibrosis, a PTPRR expression promotor, and a kit for treatment of pulmonary fibrosis.

### BACKGROUND ART

Idiopathic interstitial pneumonia (IIP) is a disease group of interstitial pneumonias of unknown etiology that share similar clinical features and classified into 6 histologic subtypes. All the subtypes are characterized by varying degrees of inflammation and fibrosis and all cause dyspnea and typical radiographic abnormalities. The 6 histologic subtypes of idiopathic interstitial pneumonia in decreasing order of frequency are usual interstitial pneumonia (UIP), nonspecific interstitial pneumonia, bronchiolitis obliterans with organizing pneumonia, interstitial lung disease (ILD) with respiratory bronchiolitis, desquamative interstitial pneumonia, and acute interstitial pneumonia.

Idiopathic pulmonary fibrosis (IPF, idiopathic fibrosing alveolitis), identified histologically as UIP described above, accounts for 50% of IIP cases. IPF is referred to as pneumonia, the role of inflammation of IPF is insignificant, and environmental, genetic, or other unknown factors are thought to initially trigger alveolar epithelial cell injury. Self-perpetuating and aberrant interstitial fibroblast and mesenchymal cell proliferation (with collagen deposition and fibrosis) are thought to account for development of clinical disease. Most patients have moderate to advanced clinical disease at the time of diagnosis and the disease deteriorates despite treatment. Currently, no specific treatment method for IPF has been proven effective. Corticosteroids and cytotoxic drugs (cyclophosphamide, azathioprine) have traditionally been administered to IPF patients empirically in an attempt to halt the progression of inflammation, but limited data support their efficacy (Non Patent Document 1).

The beneficial effects of antifibrotic agents, pirfenidone and nintedanib, have been confirmed using a general pulmonary fibrosis model, "bleomycin model". However, the pathological conditions of the "bleomycin model" do not always match those of pulmonary fibrosis, and pirfenidone and nintedanib have been confirmed to have only an effect of suppressing disease progression. Furthermore, it has been reported that in clinical trials using drugs suggested to have efficacy by the use of the "bleomycin model", no effective therapeutic effect on IPF patients has been obtained in most cases (Non Patent Document 2). Even if effects on lung diseases have been confirmed with the use of the bleomycin model, however, it cannot be currently said that the effects can be actually confirmed on pulmonary fibrosis. Moreover, lung transplantation is employed as a treatment method for pulmonary fibrosis, but is problematic in lack of donors and rejection upon transplantation. Accordingly, development of a novel treatment method for pulmonary fibrosis has been desired.

Meanwhile, mesenchymal stem cells are pluripotent progenitor cells that were isolated from the bone marrow for the first time by Friedenstein (1982) (Non Patent Document 3). The presence of the mesenchymal stem cells in various tissues including bone marrow, umbilical cord, fat, and the like has been revealed. Mesenchymal stem cell transplantation is expected as a novel treatment method for various intractable diseases (Patent Documents 1 to 4). It is currently known that cells having functions equivalent to those of mesenchymal stem cells are present among mesenchymal stromal cells of adipose tissue and fetal appendages such as placenta, umbilical cord, and egg membrane. Hence, the mesenchymal stem cells may also be referred to as mesenchymal stromal cells.

### PRIOR ART DOCUMENTS

### Patent Documents

Patent Document 1: JP 2002-506831 A
Patent Document 2: JP 2000-508911 A
Patent Document 3: JP 2012-157263 A
Patent Document 4: JP 2012-508733 A

### Non Patent Documents

Non Patent Document 1: The Merck Manual of Diagnosis and Therapy, 18th Edition, Japanese version, pp. 466-471, February 22, 2007, published by Nikkei BP
Non Patent Document 2: Timothy S. B. et al., American Journal of Respiratory and Critical Care Medicine, p. 214-222, 189(2), 2014
Non Patent Document 3: Pittenger F. M. et al., Science 284, pp. 143-147, 1999

### SUMMARY OF INVENTION

### Technical Problem

Under the above circumstances, an object of the present invention is to provide a novel therapeutic agent for pulmonary fibrosis, particularly idiopathic pulmonary fibrosis (IPF), for which currently no effective pharmacotherapy has been established.

### Solution to Problem

In order to achieve the above object, the present inventors verified therapeutic effects of mesenchymal stem cells on pulmonary fibrosis using cells collected from tissues having lesions of pulmonary fibrosis. As a result, the inventors found that mesenchymal stem cells can inhibit fibrosis. The present invention provides a novel therapeutic agent for pulmonary fibrosis. The gist of the present invention is as follows.
[1] A therapeutic agent for pulmonary fibrosis comprising mesenchymal stem cells.
[2] The therapeutic agent for pulmonary fibrosis according to [1], wherein pulmonary fibrosis is usual interstitial pneumonia (UIP) or idiopathic pulmonary fibrosis (IPF).
[3] The therapeutic agent for pulmonary fibrosis according to [1] or [2], wherein the mesenchymal stem cells are derived from an adipose tissue.
[4] The therapeutic agent for pulmonary fibrosis according to any one of [1] to [3], wherein the mesenchymal stem cells are allogenic.
[5] A therapeutic agent for pulmonary fibrosis comprising a PTPRR expression promotor.
[6] The therapeutic agent for pulmonary fibrosis according to [5], wherein the PTPRR expression promotor dephosphorylates phosphorylated ERK.
[7] A kit for treatment of pulmonary fibrosis, comprising the therapeutic agent for pulmonary fibrosis according to any one of [1] to [6], a container, and a label.

### Advantageous Effects of Invention

According to the present invention, a novel therapeutic agent for pulmonary fibrosis, a PTPRR expression promotor, and a novel kit for treatment of pulmonary fibrosis can be provided.

### BRIEF DESCRIPTION OF DRAWINGS

[Fig. 1] Fig.1 shows the effects of the therapeutic agent for pulmonary fibrosis of the present invention on the fibrosis-related gene expression (a-SMA, CDH2, Collagen 1) in cells derived from lesions of pulmonary fibrosis.
[Fig. 2] Fig.2 shows the effects of the therapeutic agent for pulmonary fibrosis of the present invention on dephosphorylation of phosphorylated ERK molecules within cells derived from lesions of pulmonary fibrosis.
[Fig. 3] Fig. 3 shows the effects of the therapeutic agent for pulmonary fibrosis of the present invention on fibrosis-related gene expression (a-SMA, CDH2, FN1) in alveolar epithelium-like cell lines.
[Fig. 4] Fig. 4 shows the histological images of the lungs of a severe combined immunodeficient mouse (pulmonary fibrosis model) to which cells derived from lesions of pulmonary fibrosis were administered.

### DESCRIPTION OF EMBODIMENTS

The therapeutic agent for pulmonary fibrosis and the kit for treatment of pulmonary fibrosis of the present invention will be described in detail.

### <Therapeutic agent for pulmonary fibrosis>

The therapeutic agent for pulmonary fibrosis of the present invention comprises mesenchymal stem cells. Mesenchymal stem cells act on the cells of involved tissues of fibrosing lungs, exerting an effect of lowering the expression of a fibrosis-related gene by cells. Moreover, administration of mesenchymal stem cells to animals with fibrosis can be expected not only to be effective at the level of cells, but also to alleviate fibrosis. The therapeutic agent for pulmonary fibrosis in the present invention may comprise, in addition to mesenchymal stem cells, other components unless the effects of the present invention are not deteriorated. Mesenchymal stem cells and other components will be described in detail as follows.

### (Mesenchymal stem cells)

The term "mesenchymal stem cells" in the present invention refers to cells being capable of differentiating into one or more cells, preferably two or more cells, and further preferably three or more cells belonging to the mesenchyme (bone cells, cardiomyocytes, cartilage cells, tendon cells, fat cells and the like), and capable of proliferating while keeping the capability. The term "mesenchymal stem cells" as used in the present invention refers to cells same as mesenchymal stromal cells and the two are not particularly differentiated. In addition, the term is simply denoted as mesenchymal cells. Examples of tissue containing mesenchymal stem cells include adipose tissue, umbilical cord, bone marrow, umbilical cord blood, endometrial, placenta, amnion, chorion, decidua, dermis, skeletal muscle, periosteum, dental sac, periodontal ligament, dental pulp, and tooth germ. For example, the term "adipose tissue-derived mesenchymal stem cells" refers to mesenchymal stem cells contained in adipose tissues, and may also be referred to as adipose tissue-derived mesenchymal stromal cells. Of these, in view of efficacy for treatment of pulmonary fibrosis, the ease of availability, and the like, adipose tissue-derived mesenchymal stem cells, umbilical cord-derived mesenchymal stem cells, bone marrow-derived mesenchymal stem cells, placenta-derived mesenchymal stem cells, and dental pulp-derived mesenchymal stem cells are preferable, adipose tissue-derived mesenchymal stem cells and umbilical cord-derived mesenchymal stem cells are more preferable, and adipose tissue-derived mesenchymal stem cells are even more preferable.

Mesenchymal stem cells in the present invention may be derived from the same species as or different species from that of a subject to be treated (test subject). Examples of the species of mesenchymal stem cells in the present invention include human, monkey, horse, cattle, sheep, pig, dog, cat, rabbit, mouse and rat, and preferably the mesenchymal stem cells are cells derived from the same species as that of a subject to be treated (test subject). The mesenchymal stem cells in the present invention may be derived from a subject to be treated (test subject); that is, isogenic (autologous) cells, or may be derived from another subject of the same species; that is, may be allogeneic cells. The mesenchymal stem cells are preferably allogeneic cells.

Mesenchymal stem cells are unlikely to cause rejection also in an allogenic test subject. Therefore, previously prepared donor cells subjected to expansion culture and then cryopreservation can be used as mesenchymal stem cells in the therapeutic agent for pulmonary fibrosis of the present invention. Accordingly, compared with a case in which autologous mesenchymal stem cells are prepared for use, mesenchymal stem cells in the present invention are more preferably allogenic in view of easy commercialization and ease of obtaining some stable effectiveness.

The term "mesenchymal stem cells" in the present invention refers to any cell population containing mesenchymal stem cells. Such a cell population comprises at least 20% or more, preferably 30%, 40%, 50%, 60%, 70%, 75%, 80%, 85%, 90%, 93%, 96%, 97%, 98% or 99% of mesenchymal stem cells.

The term "adipose tissue" in the present invention refers to tissue containing fat cells and mesenchymal stem cells including microvascular cells and the like, which can be obtained through surgical resection or suction of subcutaneous fat of mammals, for example. Adipose tissue can be obtained from subcutaneous fat. Adipose tissue is preferably obtained from animals of the same species as that of a test subject to which adipose tissue-derived mesenchymal stem cells are administered as described later. In view of administration to humans, adipose tissue is more preferably human subcutaneous fat. A donor (individual) from which subcutaneous fat is supplied may be alive or dead, however, adipose tissue to be used in the present invention is preferably tissue collected from a living individual. When adipose tissue is collected from an individual, examples of liposuction include PAL (power-assisted) liposuction, elcornia laser liposuction, and body jet liposuction. In view of maintaining cell status, preferably no ultrasonic wave is used.

The umbilical cord in the present invention is a white tubular tissue connecting between the fetus and the placenta, is composed of umbilical cord veins, umbilical cord arteries, mucous connective tissue (Wharton's Jelly), umbilical cord matrix itself, and the like, and is rich in mesenchymal stem cells. The umbilical cord is preferably obtained from animals of the same species as that of a test subject (a subject to which the agent is administered) for which the therapeutic agent for pulmonary fibrosis of the present invention is used. In view of administration of the therapeutic agent for pulmonary fibrosis of the present invention to humans, the umbilical cord is more preferably human umbilical cord.

The term "bone marrow" in the present invention refers to spongy tissue filling the bone lumen and is a hematopoietic organ. Bone marrow aspirate is present in the bone marrow, and cells existing therein are referred to as "bone marrow cells". Bone marrow cells include, in addition to erythrocytes, granulocytes, megakaryocytes, lymphocytes, fat cells and the like, mesenchymal stem cells, hematopoietic stem cells, vascular endothelial precursor cells, and the like. Bone marrow cells can be collected from human ilia, long bones, or other bones, for example.

The term "mesenchymal stem cells derived from each tissue" in the present invention such as adipose tissue-derived mesenchymal stem cells, umbilical cord-derived mesenchymal stem cells, and bone marrow-derived mesenchymal stem cells refer to any cell population containing mesenchymal stem cells derived from each tissue such as adipose tissue-derived mesenchymal stem cells, umbilical cord-derived mesenchymal stem cells, and bone marrow-derived mesenchymal stem cells, respectively. Such a cell population comprises at least 20% or more, preferably 30%, 40%, 50%, 60%, 70%, 75%, 80%, 85%, 90%, 93%, 96%, 97%, 98% or 99% of mesenchymal stem cells derived from each tissue such as adipose tissue-derived mesenchymal stem cells, umbilical cord-derived mesenchymal stem cells, and bone marrow-derived mesenchymal stem cells.

Mesenchymal stem cells in the present invention may be characterized by growth characteristics (e.g., population doubling capability or doubling time required from passages to senescence), karyotype analysis (e.g., normal karyotype; maternal or neonatal sequence), surface marker expression as determined by flow cytometry (e.g., FACS analysis), immunohistochemistry and/or immunocytochemistry (e.g., epitope detection), gene expression profiling (e.g., gene chip arrays; polymerase chain reaction such as reverse transcription PCR, real time PCR, and conventional PCR)), miRNA expression profiling, protein arrays, secretion of proteins such as cytokines (e.g., analysis of plasma clotting, ELISA, and cytokine arrays) metabolites (metabolome analysis), other methods known in the art, and the like.

### (Method for preparing mesenchymal stem cells)

Mesenchymal stem cells can be prepared by a method well-known by persons skilled in the art. A method for preparing adipose tissue-derived mesenchymal stem cells is described below as an example. Adipose tissue-derived mesenchymal stem cells may be obtained, for example, by the production method disclosed in U.S. Patent No. 6,777,231, and can be produced, for example, by a method comprising the following steps (i) to (iii):
(i) step of obtaining a cell suspension by enzymatic digestion of adipose tissue;
(ii) step of precipitating cells for re-suspension of cells in an appropriate medium; and
(iii) step of culturing cells on a solid surface and then removing cells not binding to the solid surface.

Adipose tissue washed in advance is preferably used in step (i). Washing can be performed using a physiologically compatible saline solution (e.g., phosphate-buffered saline (PBS)) and vigorously stirring for precipitation. This is performed for removing contaminants (also referred to as debris such as damaged tissue, blood, and erythrocytes) contained in adipose tissue from the tissue. Therefore, washing and precipitation are generally repeated until debris is removed overall from the supernatant. Remaining cells are present as various sized masses and are dissociated with minimum damage to the cells themselves. Hence, cell mass after washing is preferably treated with an enzyme (e.g., collagenase, dispase or trypsin) that weakens or disrupts cell-to-cell binding. The amount of and the period for treatment with such an enzyme are varied depending on conditions to be employed, but are well-known in the art. Instead of or in combination with such enzymatic treatment, cell mass can be dissociated by other treatment methods such as mechanical stirring or a method using ultrasonic energy, thermal energy or the like. In order to minimize damage to cells, enzymatic treatment alone is preferably employed. When an enzyme is used, it is desired to inactivate the enzyme using medium and the like after an appropriate period of time in order to minimize harmful effects on cells.

A cell suspension obtained in step (i) contains aggregated cell slurry or suspension, and various contaminating cells such as erythrocytes, smooth muscle cells, endothelial cells, and fibroblasts. Therefore, aggregated cells and these contaminating cells may be subsequently separated and removed. However, since these cells can be removed by adhesion and washing in step (iii) described later, such separation and removal can be omitted. When contaminating cells are separated and removed, separation and removal can be achieved by centrifugation that forcibly separates cells into a supernatant and a precipitate. The precipitate thus obtained containing contaminating cells is suspended in a physiologically compatible solvent. The suspended cells may include erythrocytes. However, the erythrocytes are removed by selection that involves adhesion thereof to the solid surface described later, a step of lysis is not always required. As a method for selectively lysing erythrocytes, for example, a method well-known in the art such as incubation in hypertonic medium or hypotonic medium using lysis with ammonium chloride can be employed. After lysis, the lysate may be separated from desired cells, for example, by filtration, centrifugal sedimentation, fractionation by density, or the like.

In step (ii), suspended cells may be washed once or multiple times successively, centrifuged, and then re-suspended in medium in order to increase the purity of mesenchymal stem cells. In addition to these treatments, cells may be separated on the basis of cell surface marker profile or cell size and the granular nature of the cells.

A medium to be used for re-suspension is not particularly limited as long as it is a medium in which mesenchymal stem cells can be cultured. Such a medium may also be prepared by adding serum, and/or one or more serum substitutes such as albumin, transferrin, fatty acid, insulin, sodium selenite, cholesterol, collagen precursor, trace element, 2-mercaptoethanol, and 3'-thiolglycerol to a basal medium. These media may be further supplemented with substances such as lipids, amino acids, proteins, polysaccharides, vitamins, growth factors, low-molecular-weight compounds, antibiotics, antioxidants, pyruvic acids, buffer, and inorganic salts, as necessary.

Examples of the above basal medium include IMDM, Medium 199, Eagle's Minimum Essential Medium (EMEM), αMEM, Dulbecco's Modified Eagle's Medium (DMEM), Ham's F12 medium, RPMI 1640 medium, Fischer's medium, MCDB201 medium and mixed media thereof.

Examples of the above serum include, but are not limited to, human serum, fetal bovine serum (FBS), bovine serum, fetal calf serum, goat serum, horse serum, porcine serum, sheep serum, rabbit serum, and rat serum. When such serum is used, it may be added in an amount of 5 v/v% to 15 v/v%, preferably 10 v/v% relative to the basal medium.

Examples of the above fatty acid include, but are not limited to, linoleic acid, oleic acid, linolenic acid, arachidonic acid, myristic acid, palmitoyl acid, palmitic acid, and stearic acid. Examples of lipids include, but are not limited to, phosphatidyl serine, phosphatidyl ethanolamine, and phosphatidyl choline. Examples of amino acids include, but are not limited to, L-alanine, L-arginine, L-aspartic acid, L-asparagine, L-cysteine, L-cystine, L-glutamic acid, L-glutamine, and L-glycine. Examples of proteins include, but are not limited to, ecotin, reduced glutathione, fibronectin and β2-microglobulin. Examples of polysaccharides include, but are not limited to, glycosaminoglycans and, of these glycosaminoglycans, particularly hyaluronic acid, and heparan sulfate. Examples of growth factors include, but are not limited to, platelet-derived growth factor (PDGF), basic fibroblast growth factor (bFGF), transforming growth factor beta (TGF-β), hepatocyte growth factor (HGF), epidermal growth factor (EGF), connective tissue growth factor (CTGF), and vascular endothelial cell growth factor (VEGF). From the view point of using adipose -derived mesenchymal stem cells obtained in the present invention for cell transplantation, it is preferable to use a (Xeno-Free) medium containing no heterologous components such as serum. As a medium, a commercially available ready-made medium for mesenchymal stem cells (stromal cells) is provided by a manufacturer such as PromoCell GmbH, Lonza, Biological Industries, Veritas, R&D Systems, Corning Inc. and Rohto Pharmaceutical Co., Ltd.

Subsequently, in step (iii), cells are cultured on a solid surface without allowing differentiation of cells in the cell suspension obtained in step (ii), using any appropriate cell medium as described above, appropriate cell density and culture conditions. In the present invention, the term "solid surface" refers to any material that enables binding and adhesion of adipose tissue-derived mesenchymal stem cells in the present invention. In a specific aspect, such a material is a plastic material treated to promote binding and adhesion of mammalian cells onto the surface. The shape of a culture vessel having a solid surface is not particularly limited, and petri dishes, flasks, and the like are used suitably. To remove unbound cells and cell fragments, cells are washed after incubation.

In the present invention, finally cells remaining bound and adhered onto the solid surface can be selected as cell populations of adipose tissue-derived mesenchymal stem cells.

To confirm that the cells thus selected are adipose tissue-derived mesenchymal stem cells in the present invention, the cells may be analyzed for surface antigens by a conventional method using flow cytometry or the like. Moreover, the cells may also be tested for their ability to differentiate into each cell lineage, and such differentiation can be performed by a conventional method.

Mesenchymal stem cells in the present invention can be prepared as described above, and may be defined as cells having the following properties of:
(1) exhibiting adhesion to plastic under culture conditions of standard medium;
(2) being positive for surface antigens CD44, CD73, and CD90, and negative for CD31 and CD45; and
(3) being capable of differentiating into bone cells, fat cells, and cartilage cells under culture conditions.

### (Cryopreservation of mesenchymal stem cells)

Mesenchymal stem cells in the present invention may be cells repeatedly cryopreserved and thawed as appropriate, as long as the cells have therapeutic effects on pulmonary fibrosis. In the present invention, cryopreservation can be performed by suspending mesenchymal stem cells in a cryopreservation solution well-known by persons skilled in the art and then cooling the resultant. Suspension can be performed by dissociating cells using a cell-dissociating agent such as trypsin, transferring cells into a cryopreservation container, treating as appropriate, and then adding a cryopreservation solution.

A cryopreservation solution may contain DMSO (Dimethyl sulfoxide) as a cryoprotectant. However, DMSO has a property of inducing differentiation of mesenchymal stem cells in addition to cytotoxicity, and thus reducing the DMSO content is preferred. Examples of an alternative to DMSO include glycerol, propylene glycol or polysaccharides, and sugar alcohols. When DMSO is used, the concentration of DMSO in a cryopreservation solution ranges from 5% to 20%, preferably 5% to 10%, and is more preferably 10%. In addition to this, the cryopreservation solution may also contain an additive disclosed in WO2007/058308. As such a cryopreservation solution, for example, a cryopreservation solution provided from BioVerde, NIPPON Genetics, ReproCELL, ZENOAQ, COSMO BIO, Kohjin Bio Co., Ltd., Thermo Fisher Scientific K.K. or the like may be used.

When the above-suspended cells are cryopreserved, the cells may be stored at a temperature between -80°C and -100°C (e.g., -80°C) using any freezer that can reach the temperatures. The type of a freezer is not particularly limited, and a program freezer may be used to control the cooling rate as appropriate in order to avoid any rapid temperature change. The cooling rate may be selected as appropriate depending on the components of a cryopreservation solution and cooling can be performed according to the instructions of the manufacturer of the cryopreservation solution.

The upper limit of the preservation period is not particularly specified, as long as cells retain their characteristics equivalent to those before freezing, after thawing of the cryopreserved cells under the above conditions. Examples of the preservation period include 1 week or more, 2 weeks or more, 3 weeks or more, 4 weeks or more, 2 months or more, 3 months or more, 4 months or more, 5 months or more, 6 months or more, 1 year or more, or periods longer than these. Cell damage can be inhibited by preserving cells at a lower temperature, and thus cells may be transferred to and then preserved in a gas phase over liquid nitrogen (about -150°C or lower and -180°C or higher). When cells are preserved in a gas phase over liquid nitrogen, preservation can be performed using a preservation container well-known by persons skilled in the art. Cells are preferably preserved in a gas phase over liquid nitrogen when the cells are preserved, for example, for 2 weeks or more, and a way of preservation is not particularly limited thereto.

Thawed mesenchymal stem cells may be cultured as appropriate until the next cryopreservation. Mesenchymal stem cells are cultured using a medium in which the above mesenchymal stem cells can be cultured without particular limitation and may also be cultured at culture temperatures ranging from about 30°C to 40°C, and at preferably about 37°C under an atmosphere of CO₂-containing air. CO₂ concentration ranges from about 2% to 5%, and is preferably about 5%. Upon culturing, after the culture reaches appropriate confluency for the culture vessel (for example, 50% to 80% of the culture vessel is occupied by cells), cells may be dissociated using a dissociating agent such as trypsin, and then seeded at appropriate cell density for a separately prepared culture vessel, and may be continuously cultured. Upon seeding of cells, examples of typical cell density include 100 cells/cm² to 100,000 cells/cm², 500 cells/cm² to 50,000 cells/cm², 1,000 to 10,000 cells/cm², and 2,000 to 10,000 cells/cm². In a specific aspect, cell density ranges from 2,000 to 10,000 cells/cm². The period required for the culture to reach appropriate confluency is preferably adjusted to range from 3 days to 7 days. During culture, medium exchange may be, as appropriate, performed as necessary.

Cryopreserved cells can be thawed by a method well-known by persons skilled in the art. For example, a method is exemplified, in which cryopreserved cells are thawed by letting the cells stand or shaking the cells within a thermostatic bath or a water bath at room temperature to 37°C and preferably 37°C.

### (Form of mesenchymal stem cells)

The mesenchymal stem cells of the present invention may be cells in any form and examples thereof include cells recovered after dissociation of cells in the culture or cells frozen in a cryopreservation solution. The use of cells prepared by subdividing cells of the same lot obtained via expansion culture into small quantities followed by cryopreservation is preferable in terms of stably obtaining similar beneficial effects, excellent handleability, and the like. Mesenchymal stem cells in a cryopreserved form are thawed immediately before use, and then directly mixed with a solution for suspension of mesenchymal stem cells, such as an infusion solution or a medium while being suspended in a cryopreservation solution. Furthermore, a cryopreservation solution is removed by a method such as centrifugation and then the cells may be suspended in a solution for suspension of mesenchymal stem cells, such as an infusion solution or a medium. Here, the term "infusion solution" in the present invention refers to a solution that is used for treating humans. Examples thereof include, but are not particularly limited to, a saline solution, the Japanese Pharmacopoeia physiological saline, 5% glucose solution, the Japanese Pharmacopoeia glucose injection, Ringer's solution, the Japanese Pharmacopoeia Ringer's solution, Ringer's lactate, Ringer's acetate, No. 1 fluid (starter solution), No. 2 fluid (fluid replacement for dehydration), No. 3 fluid (maintenance fluid), and No. 4 fluid (postoperative recovery fluid). Note that the above solutions for suspension of mesenchymal stem cells, such as infusion solutions or media may be prepared to contain other components (pharmaceutically acceptable carriers and additives) described later.

The therapeutic agent for pulmonary fibrosis of the present invention may comprise, in addition to the above mesenchymal stem cells, pharmaceutically acceptable carriers and additives according to the application and the form thereof and a standard method, as long as they do not deteriorate the effects of the present invention. Examples of such carriers and additives include, but are not limited to, tonicity adjusting agents, thickeners, saccharides, sugar alcohols, preservatives, antiseptics or antimicrobial agents, pH adjusters, stabilizers, chelating agents, oily bases, gel bases, surfactants, suspending agents, binders, excipients, lubricants, disintegrants, foaming agents, fluidizing agents, dispersants, emulsifiers, buffering agents, solubilizing agents, antioxidants, sweetening agents, acidulants, colorants, flavoring agents, and aroma chemicals or refreshing agents. Examples of typical components include the following carriers and additives.

Examples of carriers include aqueous carriers such as water and hydrous ethanol. Furthermore, examples of tonicity adjusting agents (inorganic salts) include sodium chloride, potassium chloride, calcium chloride, and magnesium chloride. Examples of polyhydric alcohols include glycerin, propylene glycol, and polyethylene glycol. Examples of thickeners include carboxyvinyl polymer, hydroxyethylcellulose, hydroxypropyl methylcellulose, methylcellulose, alginic acid, polyvinyl alcohol (complete or partially saponified product), polyvinyl pyrrolidone, and macrogol. Examples of saccharides include cyclodextrin and dextrose. Examples of sugar alcohols include xylitol, sorbitol, and mannitol (may be d-, l- or dl-isomer). Examples of preservatives, antiseptics or antimicrobial agents include dibutylhydroxytoluen, butylated hydroxyanisole, alkyldiamino ethylglycine hydrochloride, sodium benzoate, ethanol, benzalkonium chloride, benzethonium chloride, chlorhexidine gluconate, chlorobutanol, sorbic acid, potassium sorbate, trometamol, sodium dehydroacetate, methyl parahydroxybenzoate, ethyl parahydroxybenzoate, propyl parahydroxybenzoate, butyl parahydroxybenzoate, oxyquinoline sulfate, phenethyl alcohol, benzyl alcohol, biguanide compound (specific examples thereof include polihexanide hydrochloride (polyhexamethylene biguanide) and the like), and Grokiru (brand name, manufactured by Rhodia Corporation). Examples of pH adjusters include hydrochloric acid, boric acid, aminoethanesulfonic acid, epsilon-aminocaproic acid, citric acid, acetic acid, sodium hydroxide, potassium hydroxide, calcium hydroxide, magnesium hydroxide, sodium hydrogencarbonate, sodium carbonate, borax, triethanolamine, monoethanolamine, diisopropanolamine, sulfuric acid, magnesium sulfate, phosphoric acid, polyphosphoric acid, propionic acid, oxalic acid, gluconic acid, fumaric acid, lactic acid, tartaric acid, malic acid, succinic acid, gluconolactone, and ammonium acetate. Examples of stabilizers include dibutylhydroxytoluen, trometamol, sodium formaldehydosulfoxylate (rongalit), tocopherol, sodium pyrosulfite, monoethanolamine, aluminum monostearate, glyceryl monostearate, sodium hydrogen sulfite, and sodium sulfite. Examples of oily bases include vegetable oils such as olive oil, corn oil, soybean oil, sesame oil, and cotton seed oil and medium-chain triglyceride. Examples of aqueous bases include Macrogol 400. Examples of gel bases include carboxyvinyl polymer and gum substance. Examples of surfactants include polysorbate 80, hardened castor oil, glycerin fatty acid ester, and sorbitan sesquioleate. Examples of suspending agents include white beeswax and various surfactants, gum arabic, gum arabic powder, xanthan gum, and soybean lecithin. Examples of binders include hydroxyethylcellulose, hydroxypropylcellulose, hydroxypropyl methylcellulose, sodium carboxymethylcellulose, polyvinyl pyrrolidone, and polyvinyl alcohol. Examples of excipients include sucrose, lactose, starch, corn starch, microcrystalline cellulose, and light silicic anhydride. Examples of lubricants include sucrose fatty acid ester, magnesium stearate, and talc. Examples of disintegrants include low substituted hydroxypropylcellulose, crospovidone, and croscarmellose sodium. Examples of foaming agents include sodium hydrogencarbonate. Examples of fluidizing agents include sodium metasilicate aluminate and light silicic anhydride.

The therapeutic agent for pulmonary fibrosis of the present invention can be provided in various forms according to purposes, such as various dosage forms including solids, semi-solids, liquids and the like. Examples of dosage forms that can be used include solids (e.g., tablets, powders, powdered drugs, granules and capsules), semi-solids [ointments (e.g., hard ointment and soft ointment), cream pharmaceuticals and the like], liquids [e.g., lotions, extract agents, suspensions, emulsions, syrups, injection preparations (including infusion solutions, embedded injection preparations, persistent injections, and injection preparations prepared before use), dialysis agents, aerosol agents, soft capsules, and drinkable preparations], adhesive preparations, and adhesive skin patches. Moreover, the therapeutic agent for pulmonary fibrosis of the present invention can also be used in the forms of solution, milky lotion, or the like in oily or aqueous vehicles. Further, the therapeutic agent for pulmonary fibrosis of the present invention can also be applied to affected parts via spraying. The therapeutic agent for pulmonary fibrosis of the present invention can also be gelatinized or formed into a sheet and then used after spraying to an affected part. The therapeutic agent for pulmonary fibrosis of the present invention can be applied to an affected part after the above mesenchymal stem cells are formed into a sheet or a tertiary structure.

The therapeutic agent for pulmonary fibrosis of the present invention can be used by suspending or diluting the above-described mesenchymal stem cells and other components (pharmaceutically acceptable carriers and additives) in/with an infusion solution such as a saline solution, the Japanese Pharmacopoeia physiological saline, 5% glucose solution, the Japanese Pharmacopoeia glucose injection, Ringer's solution, the Japanese Pharmacopoeia Ringer's solution, Ringer's lactate, Ringer's acetate, Ringer's bicarbonate, No. 1 fluid (starter solution), No. 2 fluid (fluid replacement for dehydration), No. 3 fluid (maintenance fluid), No. 4 fluid (postoperative recovery fluid), or the like, or, a solution for suspension of mesenchymal stem cells such as cell culture medium, e.g., DMEM. Preferably, the therapeutic agent for pulmonary fibrosis can be used after suspension or dilution with physiological saline, 5% glucose solution, No. 1 fluid (starter solution), and more preferably 5% glucose solution and No. 1 fluid (starter solution). Moreover, the solution for suspension of mesenchymal stem cells may be prepared in such a manner that the solution contains in advance the above other components (pharmaceutically acceptable carriers and additives).

The therapeutic agent for pulmonary fibrosis of the present invention may also be used in such a manner that mesenchymal stem cells and a solution for suspension of mesenchymal stem cells are separately enclosed and stored in different containers and then the two may be mixed before use. Note that upon storage, the above mesenchymal stem cells and solution for suspension of mesenchymal stem cells may be in a frozen form or a refrigerated form.

When the therapeutic agent for pulmonary fibrosis of the present invention is liquid, the pH of the therapeutic agent for pulmonary fibrosis is not particularly limited as long as the pH is within pharmaceutically, pharmacologically (pharmaceutically) or physiologically acceptable range, and examples of such range include a pH range of 2.5 to 9.0, preferably a pH range of 3.0 to 8.5, and more preferably a pH range of 3.5 to 8.0. Note that when mesenchymal stem cells and a solution for suspension of mesenchymal stem cells are separately enclosed and stored in different containers, only the solution for suspension of mesenchymal stem cells is required to meet the above conditions.

When the therapeutic agent for pulmonary fibrosis of the present invention is liquid, the osmotic pressure of the therapeutic agent for pulmonary fibrosis is not particularly limited, as long as the osmotic pressure is within the range acceptable by a live body. Examples of the osmotic pressure ratio of the therapeutic agent for pulmonary fibrosis of the present invention include the ratios ranging from preferably 0.7 to 5.0, more preferably 0.8 to 3.0, and further preferably 0.9 to 1.4. The adjustment of the osmotic pressure can be carried out by a well-known method in the art with an inorganic salt, a polyhydric alcohol, a sugar alcohol, a saccharide, or the like. The osmotic pressure ratio is defined as a ratio of an osmotic pressure of a sample to 286 mOsm (osmotic pressure of an aqueous 0.9 w/v% sodium chloride solution) as prescribed in The Japanese Pharmacopeia Fifteenth Edition, and an osmotic pressure is measured referring to a method for measuring an osmotic pressure (cryoscopic method) as prescribed in The Japanese Pharmacopeia. Here, the standard solution for measuring an osmotic pressure ratio (aqueous 0.9 w/v% sodium chloride solution) is prepared by drying sodium chloride (The Japanese Pharmacopeia standard reagent) at a temperature between 500°C and 650°C for 40 to 50 minutes, and thereafter allowing it to cool in a desiccator (silica gel), accurately weighing 0.900 g of the cooled sample, and dissolving the sample in purified water to precisely make up a volume of 100 mL, or alternatively, a commercially available standard solution for measuring an osmotic pressure ratio (aqueous 0.9 w/v% sodium chloride solution) is used. Note that if mesenchymal stem cells and a solution for suspension of mesenchymal stem cells are separately enclosed and stored in different containers, only the solution for suspension of mesenchymal stem cells is required to meet the above conditions.

Examples of the route of administration of the therapeutic agent for pulmonary fibrosis of the present invention to a subject include peroral administration, subcutaneous administration, intramuscular administration, intravenous administration, intraarterial administration, intrathecal administration, intraperitoneal administration, sublingual administration, transrectal administration, transvaginal administration, transnasal administration, inhalation, transdermal administration, implant, and direct administration via spraying over or applying a sheet or the like onto the pulmonary surface. In view of the efficacy of the therapeutic agent for pulmonary fibrosis of the present invention, the routes of administration are preferably implant, intraarterial administration, intravenous administration and direct administration via spraying over or applying a sheet or the like over the pulmonary surface. In view of lowering the burden on a subject, the route of administration is more preferably intra-pulmonary arterial administration or intravenous administration, and is most preferably intravenous administration.

The dose (dosage) of the therapeutic agent for pulmonary fibrosis of the present invention can differ depending on a patient's conditions (e.g., body weight, age, symptoms, and physical condition), dosage form of the therapeutic agent for pulmonary fibrosis of the present invention, and the like. In view of having sufficient therapeutic effects on pulmonary fibrosis, a higher dose tends to be preferred. On the other hand, in view of suppressing the expression of adverse reaction, a lower dose tends to be preferred. In general, when the agent is administered to an adult, the number of cells ranges from 1 × 10³ to 1 × 10¹² cells/administration, preferably 1 × 10⁴ to 1 × 10¹¹ cells/administration, more preferably 1 × 10⁵ to 1 x 10¹⁰ cells/administration, and further preferably 5 × 10⁶ to 1 × 10⁹ cells/administration. Furthermore, the dosage per kg body weight of a patient ranges from 1 × 10 to 5 × 10¹⁰ cells/kg, preferably 1 × 10² to 5 × 10⁹ cells/kg, more preferably 1 × 10³ to 5 × 10⁸ cells/kg, and further preferably 1 × 10⁴ to 5 × 10⁷ cells/kg. Note that this amount of the agent may be considered as a single dose and administered multiple times, or this amount of the agent may be administered in divided doses.

The rate of administration of the therapeutic agent for pulmonary fibrosis of the present invention to a subject can differ depending on a patient's conditions (e.g., body weight, age, symptoms, and physical condition), the route of administration of the therapeutic agent for pulmonary fibrosis of the present invention, and the like. In general, when the agent is administered to an adult, the rate of administration ranges from 50 mL/h to 1,000 mL/h, preferably 75 mL/h to 500 mL/h, and more preferably 100 mL/h to 250 mL/h.

The temperature for administration of the therapeutic agent for pulmonary fibrosis of the present invention to a subject can differ depending on a patient's conditions (e.g., body weight, age, symptoms, and physical condition), the route of administration of the therapeutic agent for pulmonary fibrosis of the present invention, and the like. In general, the temperature for administration ranges from 4°C to 45°C, preferably 15°C to 37°C, and more preferably room temperature to 37°C.

The therapeutic agent for pulmonary fibrosis of the present invention may also be administered with 1 or 2 or more other drugs. Such a drug may be any drug that can be used as a drug for a respiratory organ, and an immunosuppressive drug. Examples thereof include dimorpholamine, doxapram hydrochloride hydrate, sivelestat sodium hydrate, pulmonary surfactant, dornase alfa, pirfenidone, interferon-γ-1b, predonisone, corticosteroids, pirfenidone (Pirespa (registered trademark)), nintedanib (Ofev (registered trademark)), angiotensin receptor blocker, cyclosporin, azathioprine, mizoribine, basiliximab, tacrolimus hydrate, gusperimus hydrochloride, mycophenolate mofetil, everolimus, and acetylcysteine. In addition, examples of a case in which the therapeutic agent for pulmonary fibrosis of the present invention is administered with 1 or 2 or more other drugs include various cases such as a case in which the therapeutic agent for pulmonary fibrosis of the present invention and other drugs are used simultaneously, a case in which either one of them is administered and then, after a lapse of a certain period of time, the other drug is administered, and a combination thereof.

The therapeutic agent for pulmonary fibrosis of the present invention can also be expressed as containing a PTPRR expression promotor. Here, PTPRR is protein tyrosine phosphatase receptor-type R, which is a type of receptor-type tyrosine phosphatase. PTPRR is an enzyme that dephosphorylates tyrosine of a protein phosphorylated by tyrosine kinase. Receptor-type tyrosine phosphatase is composed of an intracellular region having enzyme activity, a transmembrane domain, and an extracellular region. A ligand binds to the extracellular region so as to control the enzyme activity. The term "PTPRR expression promotor" in the present invention is referred to as a composition exhibiting an effect of promoting the expression of PTPRR gene and/or PTPRR protein in cells or tissues. The PTPRR expression promotor of the present invention is described as follows.

### [PTPRR expression promotor]

The PTPRR expression promotor in the present invention promotes PTPRR expression in cells or tissues, which causes pulmonary fibrosis, so as to enhance the dephosphorylation of tyrosine-phosphorylated proteins. As a result, the PTPRR expression promotor can suppress the cell proliferation and suppress tissue fibrosis. Examples of such tyrosine-phosphorylated proteins include ERK, JNK, and p38, and of these, ERK is preferable in view of having an excellent effect of suppressing pulmonary fibrosis.

The PTPRR expression promotor of the present invention preferably comprises mesenchymal stem cells having a function of acting on cells or tissues that cause pulmonary fibrosis, so as to promote the PTPRR expression. Description of mesenchymal stem cells contained in the above therapeutic agent for pulmonary fibrosis can be directly applied for mesenchymal stem cells contained in the PTPRR expression promotor of the present invention.

Examples of diseases against which the therapeutic agent for pulmonary fibrosis of the present invention is effective include usual interstitial pneumonia (UIP), idiopathic pulmonary fibrosis (IPF, idiopathic fibrosing alveolitis), nonspecific interstitial pneumonia, bronchiolitis obliterans with organizing pneumonia (organizing pneumonia of unknown etiology), interstitial lung disease (ILD) with respiratory bronchiolitis, desquamative interstitial pneumonia, acute interstitial pneumonitis (rapidly progressive form of interstitial pneumonia, Hamman-Rich syndrome), idiopathic interstitial pneumonia, drug-induced lung disease, eosinophilic lung disease and chronic eosinophilic pneumonia. Of these, the therapeutic agent for pulmonary fibrosis exhibits a significant effect on usual interstitial pneumonia (UIP), and idiopathic pulmonary fibrosis (IPF, idiopathic fibrosing alveolitis).

### <Kit for treatment of pulmonary fibrosis t>

The present invention encompasses a kit for treatment of pulmonary fibrosis, comprising mesenchymal stem cells and a solution for suspension of mesenchymal stem cells. Description given in the section of the therapeutic agent for pulmonary fibrosis can be applied for mesenchymal stem cells and a solution for suspension of mesenchymal stem cells contained in the kit of the present invention.

Moreover, the kit for treatment of pulmonary fibrosis of the present invention can be expressed as comprising the therapeutic agent for pulmonary fibrosis of the present invention, a container and a label. Examples of an appropriate container contained in the kit of the present invention include, and are not particularly limited to, cryotubes for freezing cells, bottles for solutions for suspension of mesenchymal stem cells, vials, and test tubes. These containers may also be formed of various materials such as glass, metal, plastic, or combinations thereof. The contents, specifically mesenchymal stem cells, solutions for suspension of mesenchymal stem cells, and the like, are described on the labels of these containers.

The kit of the present invention can comprise other materials desirable from commercial and users' viewpoints, such as other additives, other drugs, diluents, filters, needles, syringes, and package inserts containing directions for use.

### <Treatment method for pulmonary fibrosis and method for promoting PTPRR expression>

The present invention also encompasses a treatment method for pulmonary fibrosis, wherein mesenchymal stem cells are used. According to the treatment method of the present invention, mesenchymal stem cells suppress the gene expression that promotes pulmonary fibrosis, so that pulmonary fibrosis can be effectively treated. Furthermore, the present invention encompasses a method for promoting PTPRR expression in cells, tissues and the like, wherein mesenchymal stem cells are used. According to the promoting method of the present invention, mesenchymal stem cells act on cells and tissues, so as to be able to promote PTPRR expression effectively. Note that description given in the section of the therapeutic agent for pulmonary fibrosis can be applied to describe mesenchymal stem cells.

### EXAMPLES

The present invention will be described specifically as follows with reference to Examples and Test Examples, however, the present invention is not limited by these Examples and the like.

### 1. Preparation of adipose tissue-derived mesenchymal stem cells (Example 1)

A serum free medium (Rohto) for mesenchymal stem cells was added to commercially available cells (manufactured by Lonza, PT-5006), and then the cell suspension was centrifuged at 400 g for 5 minutes. After removal of the supernatant, the resultant was resuspended in a serum free medium for mesenchymal stem cells (Rohto), and then cells were seeded in a flask. Cells were subcultured at 37°C for several days in 5% CO₂. After few days, the culture product was washed with PBS to remove remaining blood cells and adipose tissue contained in the culture solution, thereby obtaining mesenchymal stem cells adhering to the plastic container.

The adipose tissue-derived mesenchymal stem cells thus obtained were aliquoted into centrifugation tubes, and then centrifuged at 800 g for 5 minutes, so that cell precipitates were obtained. After removal of the supernatants, an appropriate amount of a cryopreserved cell solution (STEM-CELLBANKER (ZENOAQ)) was added for suspension. The cell suspensions were aliquoted into cryotubes, preserved within a freezer at -80°C, transferred into gas phase over liquid nitrogen, and continuously preserved. Hereinafter, adipose tissue-derived mesenchymal stem cells are also referred to as ADMSC or ASC.

### 2. Effects of adipose tissue-derived mesenchymal stem cells on fibrosis-related gene expression in UIP patient-derived pulmonary fibroblasts (Example 2)

A specimen was collected from a patient diagnosed as having lesions of pulmonary fibrosis and then operated due to lung cancer. Sites (fibrosis (-)) observed to have no fibrosis and sites (fibrosis (+)) observed to have fibrosis were distinguished from each other, tissues of these sites were each cut into pieces, culture thereof was started in 15% serum-containing DMEM (manufactured by Wako) at 37°C in 10% CO₂. On each of days 1, 2, 4, and 7, medium exchange was performed after washing with PBS (manufactured by Wako). Culture was performed for 10 days in total, thereby obtaining UIP (usual interstitial pneumonia) patient-derived pulmonary fibroblasts (hereinafter, referred to as "UIP cells").

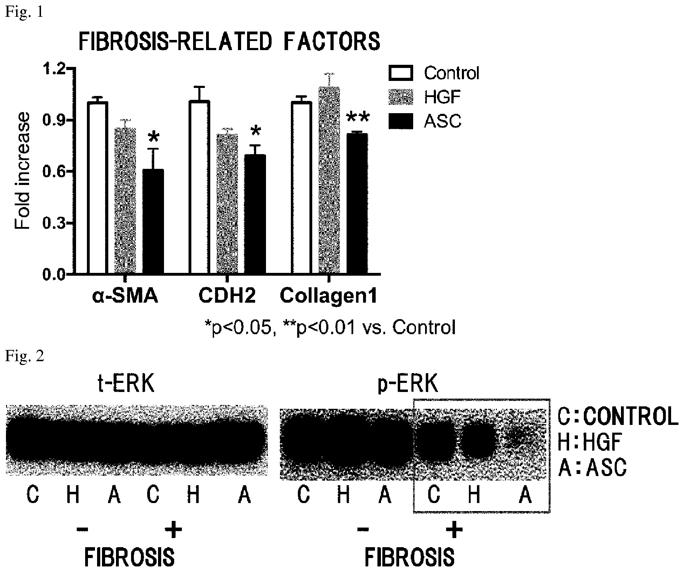

UIP cells (5.0 × 10⁴ cells/well) suspended in 15% serum-containing DMEM were seeded in each well of a 24-well plate, and then cultured overnight within a CO₂ incubator (37°C, 10% CO₂). Each culture solution was exchanged with 1% serum-containing DMEM. The above subcultured and cryopreserved human adipose tissue-derived mesenchymal stem cells (Example 1) were immersed in a thermostatic bath at 37°C to thaw the cell suspension, and then collected in a 15 mL centrifugation tube. The tube was filled with DMEM (manufactured by Wako) in such a manner that the volume reached 10 mL, and then centrifugation was performed at room temperature and 400 × g for 5 minutes (manufactured by Eppendorf, 5702). Transwell inserts (Corning, #3460) were set in a 24-well plate, to which 1% serum-containing DMEM alone (Control), 1% serum-containing DMEM containing HGF with a final concentration of 100 ng/ml, and human adipose tissue-derived mesenchymal stem cells (2.0 × 10⁵ cells/well) suspended in 1% serum-containing DMEM were added. Twenty four (24) hours after the start of co-culture, RNA of UIP cells was collected, the mRNA expression levels of fibrosis-related factors, ACTA2 (a-Smooth muscle actin; α-SMA), CDH2 (Cadherin 2) and Collagen 1, were measured by quantitative PCR. Furthermore, Western blot was performed for UIP cells. The results of quantitative PCR are shown in Fig. 1. Moreover, the results of Western blot are shown in Fig. 2.

As shown in Fig. 1, co-culture of UIP cells and human adipose tissue-derived mesenchymal stem cells caused significant decreases in expression of ACTA2 (a-SMA), CDH2 and Collagen 1 in UIP cells. The effect was more significant than that of HGF. Moreover, as shown in Fig. 2, co-culture of UIP cells and human adipose tissue-derived mesenchymal stem cells was found to cause a decrease in the level of phosphorylated ERK in UIP cells.

Furthermore, microarray analysis confirmed that the addition of human adipose tissue-derived mesenchymal stem cells causes an increase in the expression of PTPRR gene in UIP cells. This suggested a possibility such that in UIP cells, PTPRR, the expression of which has been promoted by the effect of human adipose tissue-derived mesenchymal stem cells, causes dephosphorylation of phosphorylated ERK, and thus inhibits the proliferation of cells, such as UIP cells, involving fibrosis, thereby treating fibrosis.

### 3. Effects of adipose tissue-derived mesenchymal stem cells on fibrosis-related gene expression of alveolar epithelial cell line A549 (Example 3)

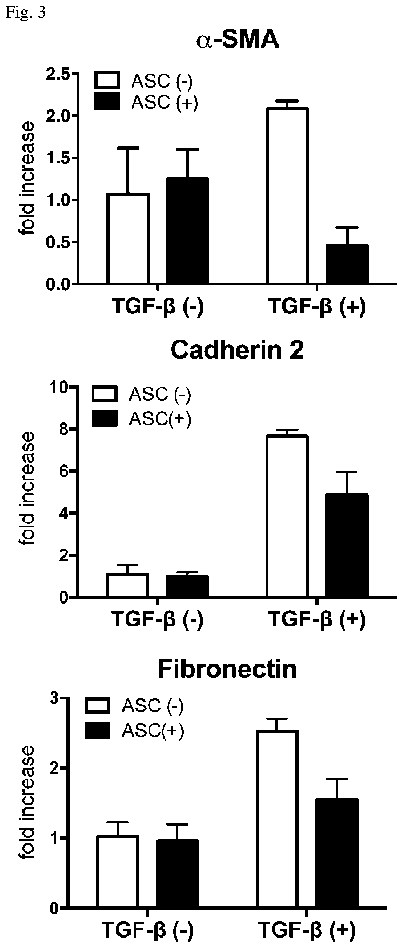

An alveolar epithelium-like cell line (A549; 5.0 × 10⁴ cells/well) suspended in 1% serum-containing DMEM was seeded in a 24-well plate, and then the 24-well plate was placed in a CO₂ incubator (37°C, 5% CO₂) to start culture. Twelve (12) hours after the start of culture, epithelial-to-mesenchymal transition was induced at the final TGF-β concentration of 100 ng/ml. After 1 day of culture, TGF-β was completely washed out using DMEM, and transwell inserts (corning, #3460) were set for an ADMSC co-culture group. Human adipose tissue-derived mesenchymal stem cells (2.0 × 10⁵ cells/well) prepared, subcultured and cryopreserved in the same manner as in Example 1 were suspended in 1% serum-containing DMEM and then the resultant was added to the transwell inserts. Twenty four (24) hours after the start of co-culture, RNA of A549 cells was collected, and then the mRNA expression levels of fibrosis-related genes, ACTA2 (a-Smooth muscle actin; α-SMA), CDH2 (Cadherin 2) and FN1 (Fibronectin 1) were measured by quantitative PCR.
The results are shown in Fig. 3.

As shown in Fig. 3, co-culture of A549 cells and human adipose tissue-derived mesenchymal stem cells caused significant decreases in expression of α-SMA, CDH2 and FN1 in A549 cells. It was thus revealed that human adipose tissue-derived mesenchymal stem cells suppress epithelial-to-mesenchymal transition (EMT) that is induced by addition of TGF-β and thought to be a cause of fibrosis, and cause decreases in the levels of fibrosis-related genes, α-SMA, CDH2 and FN1.

### 4. Preparation of fibrosis model mice using severe combined immunodeficient mice (Example 4)

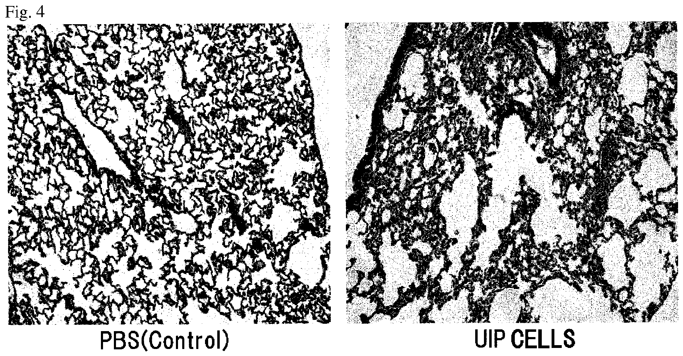

UIP cells prepared in the same manner as in Example 2 were intravenously administered to severe combined immunodeficient mice, SCID-Beige (CB17. Dg-PrkdcscidKystbg-J/CrlCrli) mice (7- to 10-week-old, female, CHARLES RIVER LABORATORIES JAPAN, INC.). Note that UIP cells were centrifuged to remove medium, 500 µL of cells (2 x 10⁶ cells) suspended in PBS (manufactured by Wako) was used for the administration. Mice to which only PBS had been administered were designated as negative control mice. Sixty-three (63) days after administration of PBS and UIP cell suspension, pathological sections of the lungs were prepared and then subjected to Masson trichrome stain. The results are shown in Fig. 4. As shown in Fig. 4, in the group to which the UIP cell suspension had been administered, clear fibrosis was confirmed and UIP-specific lesions similar to those observed in humans were observed.

### 5. Therapeutic effects of ADMSC in the fibrosis model mice (Example 5)

As in Example 4, UIP cells prepared in the same manner as in Example 2 were intravenously administered to severe combined immunodeficient mice, SCID-Beige (CB17. Dg-PrkdcscidKystbg-J/CrlCrli) mice (7- to 10-week-old, female, CHARLES RIVER LABORATORIES JAPAN, INC.). Thirty-five (35) days after administration of UIP cells, PBS alone (200 µL, manufactured by Lonza; PBS group) and human adipose tissue-derived mesenchymal stem cells (200 µL, 1.5 x 10⁶ cells; ADMSC group) prepared and subcultured in the same manner as in Example 1 were intravenously administrated respectively. Sixty-three (63) days after administration of UIP cells, pathological sections of the lungs were prepared, subjected to Masson trichrome stain, and then to histological analysis. Alleviation of fibrosis was observed in the ADMSC group compared to the PBS group, suggesting that ADMSC have a therapeutic effect on pulmonary fibrosis.

### INDUSTRIAL APPLICABILITY

According to the present invention, a novel therapeutic agent for pulmonary fibrosis, a PTPRR expression promotor and a novel kit for treatment of pulmonary fibrosis can be provided. The novel therapeutic agent for pulmonary fibrosis and the novel kit for treatment of pulmonary fibrosis of the present invention can exhibit significant therapeutic effects on pulmonary fibrosis, for which currently no effective pharmacotherapy has been established.

## Claims

1. A therapeutic agent for pulmonary fibrosis comprising mesenchymal stem cells.

2. The therapeutic agent for pulmonary fibrosis according to claim 1, wherein pulmonary fibrosis is usual interstitial pneumonia (UIP) or idiopathic pulmonary fibrosis (IPF).

3. The therapeutic agent for pulmonary fibrosis according to claim 1 or 2, wherein the mesenchymal stem cells are derived from an adipose tissue.

4. The therapeutic agent for pulmonary fibrosis according to any one of claims 1 to 3, wherein the mesenchymal stem cells are allogenic.

5. A therapeutic agent for pulmonary fibrosis comprising a PTPRR expression promotor.

6. The therapeutic agent for pulmonary fibrosis according to claim 5, wherein the PTPRR expression promotor dephosphorylates phosphorylated ERK.

7. A kit for treatment of pulmonary fibrosis, comprising the therapeutic agent for pulmonary fibrosis according to any one of claims 1 to 6, a container, and a label.
